# EUROPEAN PATENT APPLICATION

(11) **EP 2 075 001 A1**
(43) Date of publication of application: **01.07.2009**
(21) Application number: 07834499.1
(22) Date of filing: 15.10.2007
(51) Int. Cl.: A61K 31/60, A61K 31/4422, A61K 31/505, A61P 9/10, A61P 9/12, A61P 3/06

(54) **PHARMACEUTICAL COMBINATIONS FOR THE TREATMENT OF DYSLIPIDEMIA ACCOMPANIED BY HIGH BLOOD PRESSURE**

(30) Priority: 17.10.2006 MX PA06011975
(71) Applicant: WORLD-TRADE IMPORT-EXPORT, WTIE, A.G., 6320 Zug (CH)
(72) Inventor: GARCÍA ARMENTA, María Elena, C.P. 45020 Guadalajara, Jalisco (MX); ÁLVAREZ OCHOA, Victor Guillermo, C.P. 45222 Zapopan, Jalisco (MX); SANTOS MURILLO, Josefina, C.P. 44600 Zapopan, Jalisco (MX); ESPINOSA ABDALA, Leopoldo de Jésus, C.P. 45110 Zapopan, Jalisco (MX)
(74) Representative: Carvajal y Urquijo, Isabel
(86) International application number: PCT/MX2007/000118
(87) International publication number: WO 2008/048084

(57) **Abstract**

The present invention relates generally with pharmaceutical industry and with drug manufacturing pharmaceutical industry for dyslipidemia control together with arterial hypertension with a high risk of thromboembolic cardiovascular and cerebrovascular ischemic events. More specifically it deals with a composition formed by a HMG-CoA reductase inhibitor substance known as Rosuvastatin calcium combined with an antihypertensive calcium channel inhibitor known as Amlodipine besylate and an antiplatelet drug known as acetylsalicylic acid, which produce a synergic effect product, with lower delivery doses and lesser risks of adverse events.

## Description

### FIELD OF INVENTION

The present invention refers to an oral delivery pharmaceutical composition, consisting of a combination of calcium channel inhibitors or pharmaceutically acceptable salts thereof, HMG-CoA reductase inhibitors and platelet and antiplatelet drugs for treatment and control of dyslipidemia together with hypertension and with a high risk of suffering thromboembolic events.

### BACKGROUND OF INVENTION

Dyslipidemias are lipid disorders characterized by an increase in total cholesterol, LDL cholesterol, triglycerides and a reduction on HDL cholesterol.

This has been a consequence of current life style which unavoidably lead us to fast food consumption and sedentary lifestyle; this in turn has caused that in recent years overweight and obesity are a growing problem in developing and industrialized countries; overweight and obesity consequences has caused an increase in complications such as dyslipidemias, arterial hypertension, type 2 diabetes mellitus and cancer.

Our country has not been an exception since it is currently among the first positions as to overweight and obesity.

Overweight and obesity leading to dyslipidemias, arterial hypertension and type 2 diabetes mellitus, reduce life expectancy in those subjects suffering them and risks for a premature death due to cardiovascular and cerebrovascular diseases are specially increased.

A change in life style with a healthy diet and exercise has been noted to significantly prevent these risks.

At the same time, researchers have been dedicated to a task in searching new alternatives in pharmacological therapies, since although a change in life style improves and substantially reduces the risks of these diseases, in many cases it is necessary to complement this treatment with pharmacological therapies.

There is a great interest in providing for these patients better combined therapies in order to produce a synergic effect on their activity at different levels, reducing the delivery dose, reducing the side effects and specially providing a treatment compliance, since these patients unavoidably will receive the treatment all life long; however, a consideration about the interactions being present with combination delivery is of great importance, therefore our interest in developing a combined therapy which demonstrates that negative interactions are absent and on the contrary, they will provide benefit for patients.

### DETAILED DESCRIPTION OF INVENTION

The present invention relates to a combination, particularly a pharmaceutical composition comprising at least a HMG-CoA inhibitor selected from pravastatin, simvastatin, lovastatin, fluvastatin, cerivastatin, atorvastatin and rosuvastatin, at least a calcium channel inhibitor such as Amlodipine besylate and at least an antiplatelet drug known as acetylsalicylic acid, in each case a pharmaceutically acceptable salt thereof, in a single dose unit for oral delivery (capsules, tablets).

HMG-CoA reductase or also known as β-hydroxy-β-methylglutaryl-co-enzyme-A reductase inhibitors are known agents which may be used for lipid reduction including LDL cholesterol in blood.

This class of HMG-CoA reductase inhibitors has different chemical structures. Pravastatin, simvastatin, fluvastatin and rosuvastatin as well as their pharmaceutically acceptable salts are commercially available.

HMG-CoA reductase inhibitors, preferably rosuvastatin (statin), have a favorable pharmacological profile including their selective collection by liver cells, their hydrophilic nature and the absence of metabolism by isoenzyme (CYP) 3A4 from P450 cytochrome.

This last property means that the potential for pharmacological interactions mediated by CYP3A4 and consequently for side effects, is low in patients requiring a concomitant treatment with a statin and agents metabolized by CYP3A4.

Rosuvastatin is a single enantiomeric hydroxyacid which is administered as calcium salt. It has a relatively low lipophilicity. It has a high affinity for HMG-CoA reductase active site. It has a high affinity for protein C transporting predominantly hepatic organic anions.

Rosuvastatin experiences a very limited metabolism with a metabolism which mainly occurs through isoenzyme (CYP) 2C9 from P450 cytochrome, N-desmethyl rosuvastatin is the main metabolite. Rosuvastatin mainly experiences bile excretion, recovering 90% in feces with a radioactive rosuvastatin single oral dose (92% as a parent compound), removal mean life in plasma for rosuvastatin after a single oral dose of 40 mg rosuvastatin is from 18 to 24 hours.

Amlodipine is a calcium ion entry inhibitor (slow channel blocker or calcium ion antagonist) and inhibiting the transmembrane affluence from calcium ions, both in heart muscle and smooth muscle. Amlodipine action mechanism is due to a direct relaxing effect on the vessel smooth muscle. The specific mechanism whereby amlodipine relieves angina has not been fully elucidated, but amlodipine reduces the total ischemic load by the following two actions:

Amlodipine expands the peripheral arterioles, reducing the total peripheral resistance (post-load) that heart works against to. Since the heart rate is not modified, this reduction in heart work load is accompanied by a decrease in both the energy consumption and myocardium oxygen requirements.

Amlodipine action mechanism probably also involves an expansion in the main coronary arteries and arterioles, both in ischemic and normal zones.

This expansion increases the oxygen supply to myocardium in patients with coronary arterial spasm (variable angina or Prinzmetal's angina) and in acute coronary vasoconstriction episodes.

Acetylsalicylic acid induces a long term functional effect in platelets, which may be clinically detected as an extension from bleed time. This effect is mainly due to an irreversible inactivation from a key enzyme in arachidonic acid platelet metabolism. This enzyme, prostaglandin H-synthetase, is responsible of forming PGH₂, the TXA₂ prodrug. In human platelets, TXA₂ provides a mechanism for amplification of an activating signal as it is synthesized and released in response to several platelet agonists and, in turn, induces an irreversible aggregation.

In brief, therapeutics used in subjects with dyslipidemias together with systemic arterial hypertension is multiple by using pharmaceutical combinations in order to decrease the lipid level and arterial pressure, frequently in spite of the side effects which may be present, however it is our interest to use combinations presenting a double effect and minimizing adverse reactions; with this purpose tests on our combination of Rosuvastatin/Amlodipine/Acetylsalicylic acid were carried out, in order to assess the efficiency in lipid antihypertensive response indexes, as well as synergy and tolerance assessing the side effects, using lower doses in ingredients, in addition to its effect as antiplatelet drug.

Doses used in this combination are: Rosuvastatin calcium equivalent to 5 and 10 mg of rosuvastatin, Amlodipine besylate 5 mg and acetylsalicylic acid 75 mg.

### Example 1:

A randomized, double blind, crossed clinical study was carried out which objective was to assess the hypocholesterolemic, antihypertensive effect and the risk of a cardiovascular and cerebrovascular ischemic event. 100 male and female patients between 25 and 75 years old were included; lipid and total cholesterol profiles were assessed in a baseline visit, systemic arterial pressure was assessed, and baseline bleeding time, treatment compliance and adverse events were also assessed for each patient.

Baseline information showed that these 100 patients with 65 years old in average, had a total cholesterol average lipid profile of 260 mg/dl, average arterial hypertension of 190/100 mmHg and a baseline bleeding time from 1 to 6 seconds.

Patients were divided in two groups, Group 1 (n=50) received the drug independently, Rosuvastatin calcium equivalent to 5 mg of Rosuvastatin, Amlodipine besylate equivalent to 5 mg of Amlodipine and acetylsalicylic acid 5 mg, daily and along 24 weeks.

Group 2 (n=50) received a combination of a single dose unit (capsules) containing Rosuvastatin calcium equivalent to 5 mg of rosuvastatin/Amlodipine besylate equivalent to 5 mg of Amlodipine and acetylsalicylic acid 75 mg.

### Results:

40 patients completed the study within group 1 (10 cancelled the treatment because of delivery discomfort). Those 40 patients from group 1, decreased the total cholesterol levels down to an average of 180 mg/dl at the end of 24 weeks, and further they had a reduction in arterial pressure with 160/90 mmHg average, bleeding time was increased up to 12 seconds.

Group 2 completed the study with 50 patients; average reduction in cholesterol levels was 150 mg/dl, average arterial pressure reduction was 140/65 mmHg, bleeding time was noticed similar to group 1 with 12 seconds. Side effects were not present in any of the two groups, however, compliance level was full within group 2 due to an ease of administration (once a day).

Conclusions: Although group 1 showed a great improvement compared to baseline results, the fact that patients needed to take 3 different drugs makes compliance difficult unlike the administration once a day of a single unit dose which facilitates compliance with the treatment, facilitates administration with lower doses and reduces the risk of adverse events.

### Example 2:

A clinical study with 50 patients having hypercholesterolemia, hypertension and risk of cardiovascular and/or cerebrovascular ischemic events was carried out. A total baseline cholesterol of 250 mg/dl average, average arterial hypertension of 180/100 mmHg and bleeding time between 1 to 7 seconds were assessed.

Patients were divided into two groups, group 1 received Rosuvastatin calcium equivalent to 10 mg of rosuvastatin, Amlodipine besylate equivalent to 5 mg of Amlodipine and acetylsalicylic acid 75 mg, delivery was independently daily and along 24 weeks.

Group 2 received a combination of a single unit dose (capsules) containing Rosuvastatin calcium, Rosuvastatin 10 mg, Amlodipine besylate equivalent to 5 mg of Amlodipine and acetylsalicylic acid 75 mg daily along 24 weeks. Patient compliance as well as the adverse events were assessed.

### Results:

Only 17 patients completed the study within group 1 patients (n=25), from 8 patients which cancelled the treatment 2 of them had adverse effects such as severe myalgias therefore treatment was cancelled, the remaining 6 patients did not complete the full treatment due to complications in delivery.

However those 17 patients who completed the study, had an improvement in lipid profile with an average total cholesterol of 140 mg/dl, average arterial hypertension of 140/60 mmHg and bleeding time increased up to 12 seconds. Group 2 fully completed the treatment without any adverse event reported, average total cholesterol was 135 mg/dl, average arterial hypertension was 135/60 mmHg and bleeding time increased an average of 12 seconds.

Conclusions: An increase in rosuvastatin concentrations independently or in combination, has been noticed to higher reduce cholesterol levels, however, upon increasing concentrations a larger increase of adverse events is also true, therefore the use of lower concentrations in all cases is preferable, taking advantage of the synergy for activity at different levels.

### NOVELTY OF INVENTION

Having described the present invention, the contents of following claims are considered novel and therefore related with property:

## Claims

1. A synergic pharmaceutical composition **characterized by** comprising a combination of a HMG.CoA reductase inhibitor known as Rosuvastatin calcium, an antihypertensive calcium channel inhibitor known as Amlodipine besylate and an antiplatelet drug known as acetylsalicylic acid.

2. The pharmaceutical composition according to claim 1, **characterized by** comprising a synergic composition of Rosuvastatin calcium equivalent to 5 mg of Rosuvastatin, Amlodipine besylate equivalent to 5 mg of Amlodipine and acetylsalicylic acid 75 mg in a single oral unit dose (capsules or tablets).

3. The pharmaceutical composition according to claim 1 and 2, **characterized by** comprising a synergic composition of Rosuvastatin calcium equivalent to 10 mg of Rosuvastatin, Amlodipine besylate equivalent to 5 mg of Amlodipine and acetylsalicylic acid 75 mg in a single oral unit dose (capsules or tablets).

4. The pharmaceutical composition according to claims 1, 2 and 3, **characterized in that** its ingredient combination offers an improved property product requiring a lower amount of at least one ingredient and producing a synergic effect.

5. The pharmaceutical composition according to claim 1, 2, 3 and 4, **characterized in that** its ingredient combination significantly improves the serum cholesterol levels and arterial pressure, reducing the risk of cardiovascular and cerebrovascular ischemic events with a reduced risk of adverse events.

6. The pharmaceutical composition according to claims 1, 2, 3, 4 and 5, **characterized in that** calcium antagonist substances such as nimodipine, nicardipine, tercanidipine, nifedipine, varapamil and diltiazem are excluded from formulation.

7. The pharmaceutical compositions according to all the preceding claims **characterized in that** statins such as cerivastatin, pravastatin and atorvastatin are excluded from formulation.

8. The pharmaceutical compositions according to all the preceding claims **characterized** for treatment, control and prevention of dyslipidemias together with arterial hypertension and with larger risk for thromboembolic ischemic events both cardiovascular and cerebrovascular.
